# FASCICULE DE BREVET EUROPEEN

(11) **EP 2 459 083 B1**
(45) Date de publication et mention de la délivrance du brevet: **15.05.2013**
(21) Numéro de dépôt: 10752859.8
(22) Date de dépôt: 30.07.2010
(51) Int. Cl.: A61B 17/17, A61F 2/08

(54) **SYSTEME ANCILLAIRE CHIRURGICAL POUR LA PLASTIE EN FIXATION TRANSVERSALE DU LIGAMENT CROISE ANTERIEUR (LCA) DU GENOU**
CHIRURGISCHES HILFSSYSTEM FÜR DIE ERSATZPLASTIK DES VORDEREN KREUZBANDES MIT TRANSVERSALER FIXATION
SURGICAL ANCILLARY SYSTEM FOR TRANSVERSE FIXATION RECONSTRUCTION OF THE ANTERIOR CRUCIATE LIGAMENT (ACL) OF THE KNEE

(30) Priorité: 31.07.2009 FR 0903793
(43) Date de publication de la demande: 06.06.2012
(73) Titulaire: L.A.R.S. - laboratoire d'application et de Recherche Scientifique, 21560 Arc-sur-Tille (FR)
(72) Inventeur: BRULEZ, Bernard, F-52400 Bourbonne les Bains (FR); LABOUREAU, Jacques-Philippe, F-06530 Le Tignet (FR); BERBEY, Gérard, F-21910 Barges (FR)
(74) Mandataire: Oudin, Stéphane
(86) Numéro de dépôt international: PCT/FR2010/000558
(87) Numéro de publication internationale: WO 2011/012784

(56) Documents cités:
- WO-A1-00/45725
- US-A- 5 688 284
- US-A1- 2004 199 163
- US-A1- 2005 033 301
- US-A1- 2005 038 426
- US-A1- 2007 276 392

## Description

L'invention concerne un système ancillaire chirurgical pour la plastie en fixation transversale du ligament croisé antérieur (LCA) du genou.

La technique chirurgicale habituelle de fixation des ligaments artificiels ou des reconstructions ligamentaires biologiques dans le cadre d'une plastie du LCA consiste à percer deux tunnels osseux, traversants, prévus dans le prolongement l'un de l'autre, respectivement dans le fémur et dans le tibia, puis à insérer dans ces tunnels osseux un ligament prothétique. Le ligament prothétique est ensuite bloqué dans les tunnels osseux au moyen de vis à interférence fixées frontalement dans le fémur et dans le tibia, aux extrémités du ligament prothétique. Pour permettre l'insertion et la mise en place dans les tunnels osseux du ligament prothétique, les extrémités de ce dernier sont munies de fils de traction. Un des fils de traction est inséré dans les tunnels osseux afin de permettre la traction dans les tunnels osseux du ligament prothétique.

Cette technique est aujourd'hui remise en cause par les chirurgiens. En effet, elle s'avère non seulement traumatisante pour le patient qui subit deux percements osseux traversants, mais de plus, on a pu observer parfois un glissement de la vis à interférence sur le ligament prothétique au niveau de l'os du fémur.

Les chirurgiens ont donc développé une nouvelle technique d'implantation des ligaments avec un système de fixation transversale, dans lequel le tunnel fémoral est un trou borgne dans lequel débouche un tunnel latéral, également borgne. Le tunnel fémoral reçoit le ligament prothétique qui est inséré par poussée. Une fois en position, l'extrémité fémorale du ligament prothétique est fixée par l'intermédiaire d'une vis de blocage logée dans le tunnel latéral, et permettant ainsi une fixation en suspension, et éventuellement en compression, du ligament prothétique. Cette nouvelle technique de fixation des ligaments pour la plastie ligamentaire, autogène ou prothétique, offre, outre une meilleure résistance du ligament à la traction, l'avantage d'un traumatisme moindre pour le patient puisqu'il n'y a plus de percement de tunnel débouchant dans l'os du fémur et qu'en outre les diamètres des tunnels sont plus petits qu'antérieurement. Enfin, cette configuration améliore de surcroît la colonisation fibroblastique des prothèses biologiques ainsi fixées.

On connait, notamment par l'intermédiaire des publications US 2005/0 033 301, US 2004/0 199 163 et US 5,688,284, des systèmes ancillaires chirurgicaux permettant la plastie en fixation transversale du ligament croisé antérieur du genou au moyen de ligaments autogènes à tracter dans un tube fémoral prévu à cet effet dans l'articulation.

Toutefois, il n'existe pas à ce jour de système ancillaire chirurgical spécifiquement conçu pour la réalisation de cette intervention de réparation du LCA selon la technique de fixation transversale, en poussant le ligament dans le tunnel fémoral, et qui permette au chirurgien de pratiquer cette intervention en toute sécurité, aisément et de façon précise et fiable.

La présente invention a donc pour objet de fournir un système ancillaire chirurgical adapté à cette nouvelle technique de fixation transversale, au moyen duquel le ligament peut être poussé.

A cet égard, l'invention a pour objet un système ancillaire chirurgical notamment pour la plastie en fixation transversale du ligament croisé antérieur du genou, remarquable en ce qu'il est pourvu d'au moins une platine comportant au moins :
- un stabilisateur destiné à définir au moins un point d'ancrage fixe de la platine sur le genou,
- des premiers moyens de guidage d'axe longitudinal A1, aptes à recevoir au moins un premier outil chirurgical et à guider le déplacement rectiligne du premier outil chirurgical selon l'axe longitudinal A1,
- des seconds moyens de guidage d'axe transversal A3, aptes à recevoir au moins un second outil chirurgical et à guider le déplacement rectiligne du second outil chirurgical selon l'axe transversal A3,
les premiers et seconds moyens de guidage étant agencés pour que les axes longitudinal A1 et transversal A3 soient sécants entre eux, en ce que l'un au moins desdits premier et second outils chirurgicaux est un guide d'insertion apte à recevoir un ligament prothétique replié autour de son extrémité pour le pousser dans un tube fémoral, ledit guide d'insertion comportant une tige dont au moins une première portion a une section biconcave, les concavités latérales de ladite première portion étant aptes à recevoir ledit ligament prothétique allongé le long de ladite tige.

On comprend bien l'avantage de ce système ancillaire chirurgical qui permet au chirurgien de réaliser le tunnel fémoral au moyen des premiers moyens de guidage, puis la visée et le percement du tunnel latéral dans le tibia avec la certitude de déboucher dans le tunnel fémoral. Le système ancillaire chirurgical selon l'invention permet ensuite au chirurgien de mettre en place le ligament prothétique, autogène ou artificielle, en le poussant dans le tunnel fémoral et de le verrouiller en suspension dans le tibia et dans le fémur.

Les premiers et seconds moyens de guidage sont agencés pour que l'axe longitudinal A1 et l'axe transversal A3 soient sensiblement perpendiculaires entre eux et coplanaires ce qui permet d'augmenter la robustesse et la stabilité du système ancillaire chirurgical et donc la fiabilité de l'opération.

La platine peut être pourvue, en une première extrémité, d'un bras latéral comportant les premiers moyens de guidage et, en une seconde extrémité, d'une tête comportant les seconds moyens de guidage. La tête et/ou le bras latéral sont de préférence amovibles et fixés sur la platine par des moyens d'assemblage rendant le système ancillaire chirurgical facile à utiliser, à démonter et à nettoyer.

De manière avantageuse, la tête comporte au moins un premier et un second orifices respectivement d'axes transversaux A2 et A3, parallèles entre eux, l'axe transversal A2 étant décalé longitudinalement par rapport à l'axe transversal A3, le premier orifice étant agencé pour porter le stabilisateur, le second orifice définissant au moins en partie les seconds moyens de guidage.

Le premier orifice est de préférence prévu au delà du second orifice par rapport à la seconde extrémité ce qui permet d'assurer la stabilité du système ancillaire chirurgical pendant l'opération.

De manière préférentielle, le système ancillaire chirurgical comporte des moyens de verrouillage couplés à au moins l'un des premiers, seconds moyens de guidage et agencés pour bloquer en position respectivement le premier, second outil chirurgical. Les premier et second outils chirurgicaux peuvent ainsi être déplacés indépendamment l'un de l'autre, de manière rectiligne, puis bloqués simultanément ou successivement à la platine.

Les moyens de verrouillage comportent avantageusement au moins une rainure de guidage définissant un logement parallélépipédique et l'un au moins des premier, second outils chirurgicaux a au moins une section parallélépipédique agencée pour s'emboiter dans la rainure de guidage, renforçant le blocage du premier, second outil chirurgical par les moyens de verrouillage.

De manière avantageuse, l'extrémité libre de la tige du guide d'insertion présente une découpe concave apte à engager un ligament prothétique plié autour de cette découpe concave, la découpe concave étant de préférence en forme de demi-lune. La section biconcave facilite l'insertion du ligament prothétique qui peut ainsi être rabattu, de part et d'autre du guide d'insertion avant d'être poussé dans le tunnel fémoral. La découpe concave, par exemple en forme de demi-lune, reçoit l'extrémité du ligament prothétique lors de son insertion dans le tunnel fémoral et assure son bon maintien. De plus, elle laisse un passage libre pour la mise en place dans le tunnel latéral d'une vis de fixation.

Le guide d'insertion comporte avantageusement, en plus de la première portion, au moins une troisième section de section plate, dont les plats sont aptes à recevoir le ligament prothétique allongé le long de la tige.

De manière préférée, l'extrémité libre de la troisième section est pourvue de la découpe concave.

L'un au moins des premier et second moyens de guidage est couplé à au moins une butée agencée pour limiter le déplacement respectivement transversal, longitudinal du premier, second outil. Cette butée permet ainsi de garantir que le forage des tunnels ne se prolonge pas au delà de l'intersection prévue entre les deux tunnels quelle que soit la force appliquée sur la mèche de perçage. Le tunnel latéral est donc borgne ce qui limite le traumatisme subit par le patient.

La platine et le bras latéral peuvent chacun avoir une forme globalement parallélépipédique. De plus le bras latéral est avantageusement fixé sensiblement perpendiculaire à la platine. Cette construction particulière confère robustesse et fiabilité au système ancillaire chirurgical. La platine peut également être ajourée pour limiter le poids du système ancillaire chirurgical et augmenter sa maniabilité.

Le système ancillaire chirurgical est de préférence au moins en partie réalisé en acier inoxydable facilitant son utilisation et augmentant sa durée de vie.

D'autres avantages et caractéristiques ressortiront mieux de la description qui va suivre d'un exemple de réalisation donné à titre non limitatif d'un système ancillaire chirurgical notamment pour la plastie du LCA selon l'invention, en référence aux dessins annexés sur lesquels :
- la figure 1 est une vue de face en éclaté d'un système ancillaire chirurgical selon l'invention ;
- les figures 2A et 2B sont de vues de coté respectivement de la platine portant la tête et du bras latéral du système ancillaire chirurgical de la figure 1 selon les flèches D et E de la figure 1 ;
- les figures 3A et 3B sont respectivement une vue de face et une vue de coté d'un mode de réalisation du guide d'insertion de ligament, le guide d'insertion étant représenté respectivement avec un manche et seul ;
- la figure 4 est une vue de face en situation sur un genou d'un patient du système ancillaire chirurgical de la figure 1 représenté dans une configuration de percement du tunnel latéral ;
- la figure 5 est une vue de face en situation sur un genou d'un patient du système ancillaire chirurgical de la figure 1 représenté dans une configuration de mise en place du ligament prothétique.

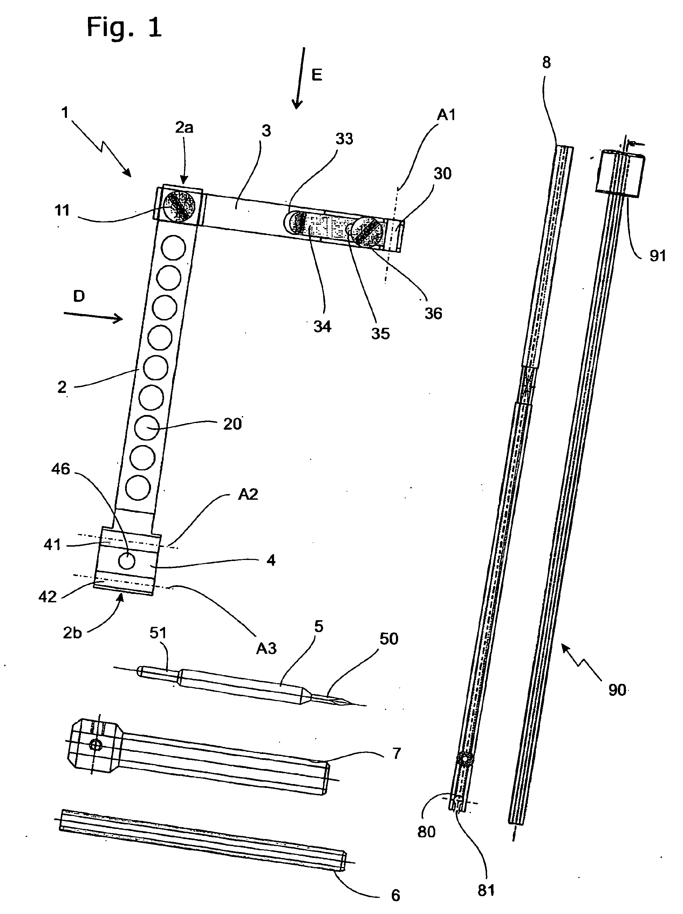

En référence à la figure 1, le système ancillaire chirurgical 1 selon l'invention comporte une platine 2, un bras latéral 3 et une tête 4. En référence à cette figure, le système ancillaire chirurgical 1 peut être équipé de différents outils comme par exemple un stabilisateur 5, un tube de guidage 6, une butée de perçage 7, un viseur transversal 8 de mise en place d'une broche et un tube passe broche 9. D'autres outils pouvant équiper le système ancillaire chirurgical 1 seront décrits plus loin. Le système ancillaire chirurgical 1 comporte des premiers moyens de guidage 30 d'axe longitudinal A1 et des seconds moyens de guidage 42 d'axe transversal A3.

La platine 2 a une forme parallélépipédique robuste et est ajourée par des évidements 20 permettant d'alléger sa structure et de faciliter sa préhension, sans toutefois la fragiliser. La platine 2 est couplée en sa première extrémité 2a, de manière démontable, à un bras latéral 3 de forme parallélépipédique. Dans une autre variante de réalisation non représentée, le bras latéral peut être fixé à demeure à la platine sans pouvoir être démonté. Le bras latéral et la platine peuvent également être formés d'une pièce unique.

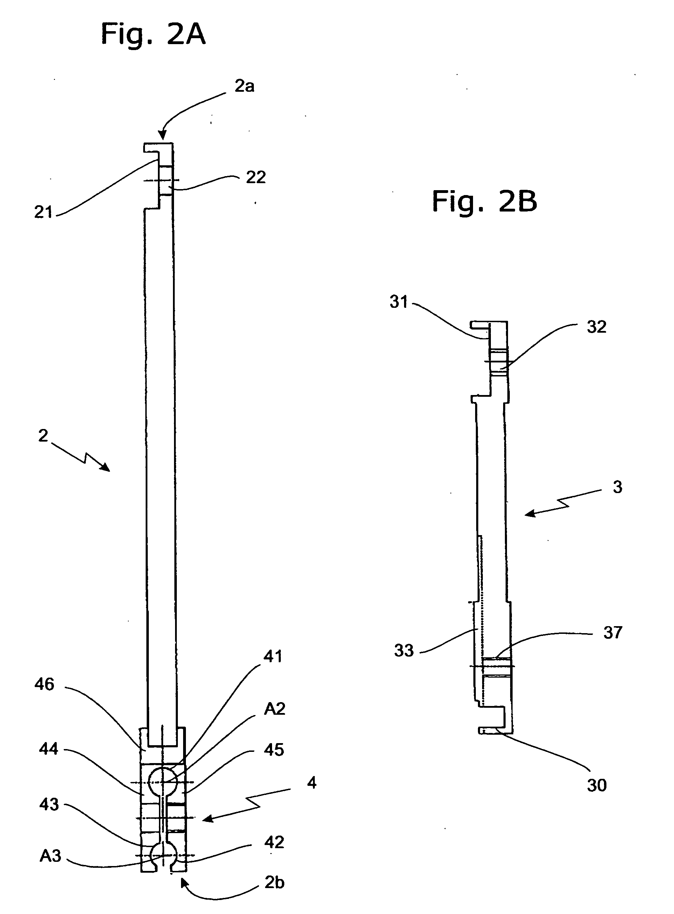

Dans cet exemple, le bras latéral 3 est solidarisé à la platine 2 de manière à lui être sensiblement perpendiculaire. A cet effet, la platine 2 et le bras latéral 3 sont respectivement pourvus d'une première et d'une seconde encoches de montage 21 et 31 visibles sur les figures 2A et 2B et traversant respectivement la platine 2 et le bras latéral 3. Les première et seconde encoches de montage 21 et 31 sont de forme rectangulaire. La première encoche de montage 21 a des dimensions inférieures à la section du bras latéral 3 permettant l'emboitement du bras latéral 3 dans la première encoche de montage 21. De même, la seconde encoche de montage 31 a des dimensions inférieures à la section de l'embase 2 permettant l'emboîtement de l'embase 2 dans la seconde encoche de montage 31. Une fois les première et seconde encoches de montage 21, 31 emboitées l'une dans l'autre, la platine 2 et le bras latéral 3 sont solidarisés l'un à l'autre au moyen d'une vis 11 traversant un orifice lisse 22 prévu dans la platine 2 en regard de la première encoche de montage 21, et vissée dans un orifice fileté 32 prévu dans le bras latéral 3 en regard de la seconde encoche de montage 31.

A l'opposé de la première encoche de montage 31, le bras latéral 3 comporte un guide longitudinal formé par une rainure de guidage 30 traversant le bras latéral 3. Cette rainure de guidage 30 a une section parallélépipédique, par exemple carrée et définit un axe longitudinal A1 (Cf. figure 1). La rainure de guidage 30 est apte à recevoir des outils chirurgicaux de section diverses. Elle est néanmoins particulièrement bien adaptée pour recevoir des outils chirurgicaux de section parallélépipédique et en particulier carrée. Le bras latéral 3 comporte une rainure de verrouillage 33 orientée sensiblement perpendiculairement à la rainure de guidage 30. Un loquet 34 est inséré dans la rainure de verrouillage 33 dans laquelle il peut être déplacé. Ce loquet 34 est traversé par une lumière 35 dans laquelle circule l'axe d'une vis de verrouillage 36 permettant de bloquer le loquet 34 dans le fond de la rainure de verrouillage 33. La vis de verrouillage 36 est vissée dans un orifice taraudé 37 (Cf. figure 2B) prévu dans le bras latéral 3. Le guide d'insertion 9, ou tout autre outil chirurgical adapté, peut être placé dans la rainure de guidage 30, déplacé de manière rectiligne selon l'axe longitudinal A1 et bloqué par la pression appliquée par le loquet 34, lui-même bloqué par la vis de verrouillage 36. Le guide d'insertion 9 peut ainsi être bloqué dans une position choisie par rapport au bras latéral 3. Le loquet 34, la vis de verrouillage 36 et la rainure de guidage 30 forment au moins en partie les moyens de verrouillage.

La deuxième extrémité 2b de la platine 2 est pourvue d'une tête 4. Dans l'exemple illustré, la tête 4 est fixée de manière permanente à la platine 2, par exemple par collage, soudure, emmanchement en force ou tout autre moyen adapté. A cet effet, la platine 2 et la tête 4 sont pourvues de formes complémentaires formant tenon et mortaise représentées sur la figure 2A. Dans une autre variante de réalisation, la tête et la platine sont formées d'une pièce unique. Dans encore une autre variante de réalisation non représentée, la tête peut être fixée de manière démontable sur la platine, par exemple d'une façon similaire à la fixation du bras latéral sur la platine.

La tête 4 comporte un premier et un second guide latéral se présentant sous la forme d'un premier et d'un second orifices 41, 42, traversant la tête 4. Les premier et second orifices 41, 42 sont destinés à recevoir des outils chirurgicaux. Ils sont disposés sensiblement parallèlement entre eux et ont respectivement un axe transversal A2 et un axe transversal A3. Les diamètres des premier et second orifices 41, 42 sont de préférence identiques pour permettre l'interchangeabilité des outils. Le premier orifice 41 est prévu au delà du second orifice 42 par rapport à la seconde extrémité 2b. L'axe transversal A2 et l'axe transversal A3 sont confondus avec le plan défini par la platine 2 et le bras latéral 3. Les premier et second orifices 41, 42 sont reliés entre eux par une fente 43, débouchante, et délimitant deux portions 44, 45 distinctes de la tête 4 reliées par une zone de jonction 46. Les diamètres des premier et second orifices 41, 42, peuvent ainsi être resserrés en rapprochant les deux portions 44, 45 l'une de l'autre, par déformation élastique de la zone de jonction 46 préservée à l'extrémité de la fente 43. Ce rapprochement peut être obtenu au moyen d'une vis non représentée, logée dans un trou 46 prévu dans les portions 44, 45, la vis traversant librement la partie lisse du trou 46 prévue dans l'une des portions 44, 45 et se vissant dans la partie taraudée du trou 46 prévue dans l'autre portion 45, 44.

Le stabilisateur 5 illustré sur la figure 1 a une forme cylindrique et est pourvue, en une première extrémité, d'une pointe 50 et, en une seconde extrémité, d'une forme arrondie 51. Le stabilisateur 5 est destiné à être inséré le long de l'axe transversal A2 dans le premier orifice 41 dans lequel il est bloqué en position par le rapprochement des portions 44, 45 l'une vers l'autre. Comme décrit plus loin, le stabilisateur 5 est utilisé pour définir un point d'ancrage fixe dans l'articulation à opérer. Dans une autre variante de réalisation non représentée, le stabilisateur est pourvu de deux pointes définissant deux points d'ancrage dans l'articulation. Le système ancillaire chirurgical peut également être pourvu de deux stabilisateurs distants, par exemple l'un étant destiné à s'ancrer dans le tibia, l'autre étant destiné à s'ancrer dans le fémur. Dans encore une autre variante de réalisation non représentée, le stabilisateur et la platine sont formés d'une pièce unique.

Le tube de guidage 6 a une forme cylindrique creuse et est destiné à être inséré le long de l'axe transversal A3 dans le second orifice 42 dans lequel il est bloqué en position par le rapprochement des portions 44, 45 l'une vers l'autre. Le tube de guidage 6 sert notamment à guider une mèche de perçage. Il peut être couplé à une butée de perçage 7, emmanchée sur le tube de guidage 6, et délimitant la course de la mèche de perçage. Ainsi, la profondeur du tunnel latéral foré dans le fémur peut être ajustée précisément en fonction de la position du tunnel fémoral préalablement foré dans le tibia et le fémur de manière à faire coïncider leurs extrémités. Ainsi, le tunnel latéral peut être prévu non débouchant ce qui limite le traumatisme de l'invention. Dans une variante de réalisation non représentée, le tube de guidage est lui-même pourvu d'une butée.

Le viseur transversal 8 se présente sous la forme d'un tube comportant deux portions cylindriques séparées par une portion de section carrée. Une des extrémités du viseur transversal 8 comporte un orifice de visée 80, traversant, prolongé jusqu'à l'extrémité du viseur transversal 8 par une fente d'accès 81. Le viseur transversal 8 est destiné à être positionné dans la rainure de guidage 30 selon l'axe longitudinal A1 pour pouvoir être inséré dans le tunnel fémoral. En fin d'insertion dans le tunnel fémoral, l'orifice de visée 80 est positionné en face du tunnel latéral de manière à caler le viseur transversal 8 dans une position prédéterminée.

Le tube passe broche 9 est utilisé pour la mise en place d'une broche dans le tunnel fémoral. Le tube passe broche 9 comporte un tube 90 de forme cylindrique creuse, prolongé par une extrémité d'un diamètre supérieur formant un épaulement 91.

D'autres outils peuvent équiper le système ancillaire chirurgical 1 comme notamment une broche cylindrique pourvue en une première extrémité d'une pointe trocart et en une seconde extrémité d'une pointe mousse. D'autres outils seront décrits plus loin.

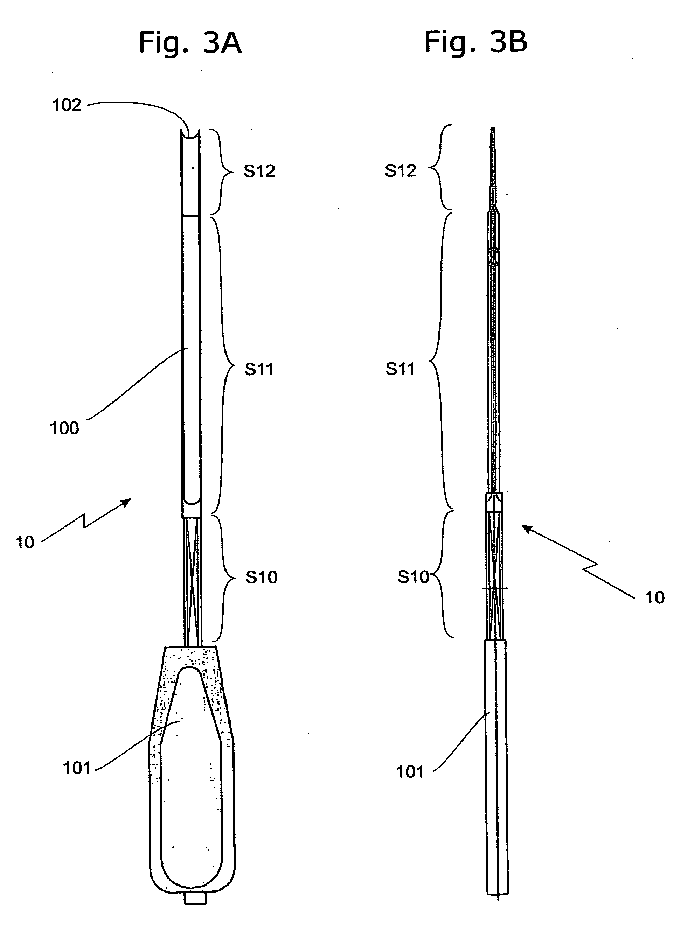

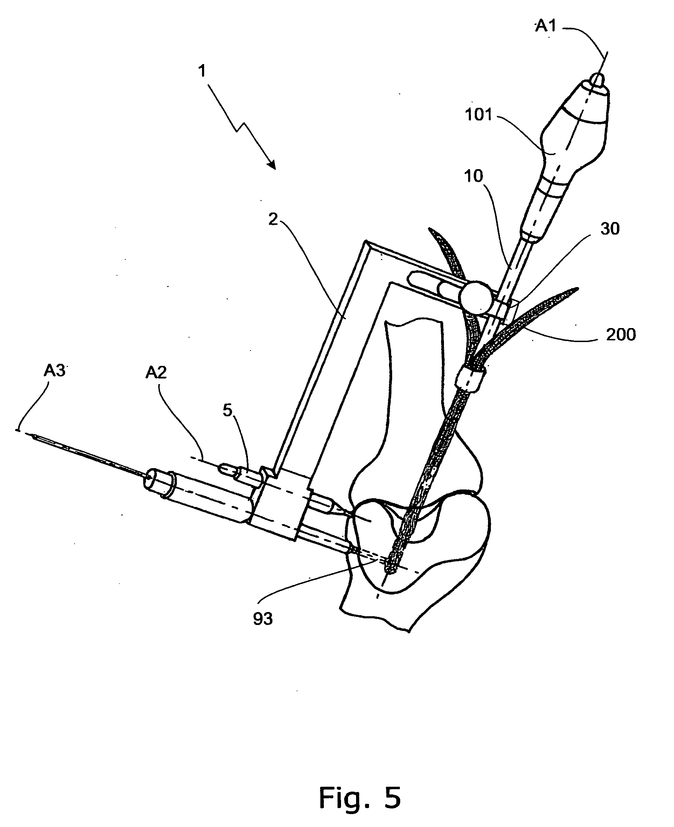

En référence aux figures 3A et 3B, le guide d'insertion 10 comporte une tige 100 présentant différentes sections, dont une première portion S11 de section biconcave, une seconde portion S10 de section sensiblement carrée, et une troisième portion S12 de section plate. Les concavités latérales de la portion S11 sont aptes à recevoir le ligament prothétique allongé le long de la tige 100. De même, les plats de la troisième portion S12 sont aptes à recevoir le ligament prothétique allongé le long de la tige 100. De plus, la tige 100, comporte en une de ses extrémités une découpe concave 102, par exemple en forme de demi-lune, et apte à engager un ligament prothétique plié autour de cette découpe concave 102. La découpe concave 102, est prévue à l'extrémité libre de la troisième portion S12 de section plate. Comme détaillé plus loin en référence à la figure 5, le ligament prothétique replié autour de la forme concave 102 et allongé dans les concavités latérales, peut ainsi être inséré dans le tunnel fémoral de manière fiable et sans risquer d'être endommagé. De plus, la découpe concave 102 permet, tout en ayant le guide d'insertion 10 toujours en place dans le tunnel fémoral, de solidariser l'extrémité fémorale du ligament prothétique au fémur au moyen d'une vis de blocage, sans que cette vis de blocage ne vienne interférer avec le guide d'insertion 10. Le guide d'insertion peut ainsi être facilement extrait du tunnel fémoral après fixation de l'extrémité fémorale du ligament prothétique. Comme illustré par la figue 3A, la tige 100 peut être fixée à un manche 101 par tout moyen adapté tel que par exemple l'emmanchement en force, le collage. La tige 100 pourvue du manche 101 est ainsi plus aisée à manipuler.

Le système ancillaire chirurgical 1 peut être, en tout ou partie réalisé en acier inoxydable.

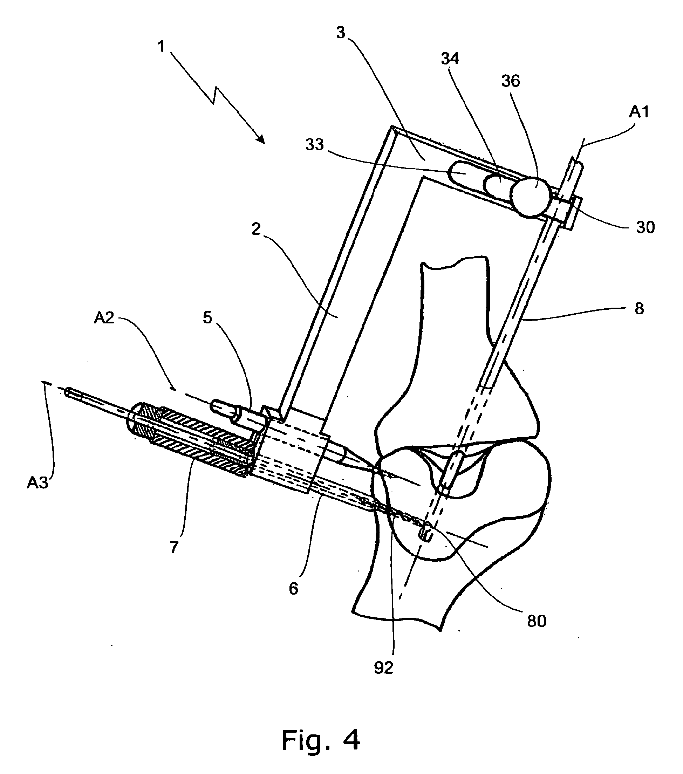

On décrira maintenant, en référence aux figures 4 et 5, le mode d'utilisation du système ancillaire chirurgical 1 selon l'invention.

Dans un premier temps, au moyen d'une mèche de perçage (non représentée), on fore un tunnel fémoral au travers du tibia et du fémur. La profondeur de ce tunnel fémoral est limité au moyen d'une butée qui permet d'obtenir un tunnel fémoral traversant le tibia et borgne dans le fémur. Comme illustré par la figure 4, on place le viseur transversal 8 le long de l'axe longitudinal A1, dans la rainure de guidage 30 du bras latéral 3. Le viseur transversal 8, est maintenu en position par l'intermédiaire de sa section carrée bloquée entre le loquet 34 et la rainure de guidage 30. L'ensemble formé du loquet 34 et du viseur transversal est bloqué au moyen de la vis de verrouillage 36. Dans cette position, l'axe de l'orifice de visée 80 du viseur transversal 8 coïncide avec l'axe transversal A3 du second orifice 42. A l'aide du système ancillaire chirurgical 1, on emmanche le viseur transversal 8 dans le tunnel fémoral. Les tronçons du tunnel fémoral situés dans le tibia et dans le fémur sont alors alignés l'un par rapport à l'autre et le viseur transversal 8 est ainsi sensiblement parallèle à la platine 2.

Dans un deuxième temps, toujours en référence à la figure 4, on emmanche le stabilisateur 5, le long de l'axe transversal A2, dans le premier orifice 41, jusqu'à ce que le stabilisateur 5 s'ancre dans le fémur de manière à définir un point d'ancrage fiable qui servira de repère fixe tout au long de l'opération. Ensuite, on place le tube de guidage 6 dans le second orifice 42 et on emmanche la butée de perçage 7 sur le tube de guidage 6. Au moyen d'une mèche de perçage 200, guidée à l'intérieur du tube de guidage 6, on vient percer le tunnel latéral le long de l'axe transversal A3. Le déplacement de la mèche de perçage 92 est limité par la butée de perçage 7 de sorte que le tunnel latéral foré soit borgne et que son extrémité débouche dans l'extrémité du tunnel fémoral déjà percé. Le viseur transversal 8 et la mèche de perçage 92 peuvent être découplés du système ancillaire chirurgical 1.

Dans un troisième temps, en référence à la figure 5, un ligament prothétique 200 est positionné sur le guide d'insertion 10 de manière à être replié autour de la découpe concave 102 de son extrémité et à être allongé, de part et d'autre de la tige 100, dans les concavités latérales de sa section biconcave S11. Le guide d'insertion 10 est positionné dans l'alignement de l'axe longitudinal A1 dans la rainure de guidage 30 et emmanché dans le tunnel fémoral en poussant le ligament prothétique 200 jusqu'à atteindre l'extrémité borgne du tunnel fémoral. Pendant cette étape, le système ancillaire chirurgical 1 est maintenu en position de manière fiable par rapport au genou au moyen du stabilisateur 5 toujours en place au niveau du fémur.

Dans un quatrième temps, toujours en référence à la figure 5, on insère une vis de blocage 92 par le second orifice 42 de manière à bloquer l'extrémité fémorale du ligament prothétique 200 au fond du tunnel fémoral. La découpe concave 102 permet à la vis de blocage 92 de ne pas interférer avec le guide d'insertion 10 et de pouvoir traverser le tunnel fémoral, fixant ainsi de manière efficace l'extrémité fémorale du ligament prothétique 200 dans le fémur.

Dans un cinquième temps (non illustré), après fixation de l'extrémité fémorale du ligament prothétique 200, le guide d'insertion 10 est retiré du tunnel fémoral. Les moyens utilisés pour visser la vis de blocage sont retirés du tunnel latéral et l'ensemble du système ancillaire chirurgical 1 peut être éloigné du genou. Les extrémités libres du ligament prothétiques 200 peuvent être fixées au tibia, par exemple au moyen d'une vis à interférence ou de tout autre moyen adapté.

Le fait que le ligament prothétique 200 puisse être poussé dans le tunnel fémoral, et non tiré comme dans la technique préalablement connue, permet de réaliser un tunnel fémoral et un tunnel latéral de diamètre plus petits qu'actuellement. Le traumatisme subit par le patient est ainsi réduit et les forces de perçage nécessaires limitées. De plus, la colonisation fibroblastique est améliorée dans ces tunnels de diamètres inférieurs. Le système ancillaire chirurgical 1 selon l'invention permet de réaliser des plasties offrant une meilleure résistance à la traction. En effet, dans la configuration de pose, le ligament prothétique 200 mis en place selon l'invention est rectiligne. Le système ancillaire chirurgical 1 selon l'invention rend donc possible une position du genou dans laquelle le ligament prothétique 200 ne subit pas d'incurvation préjudiciable à sa durée de vie.

Enfin, il va de soi que l'Homme de l'art pourra apporter des aménagements de taille, de forme et de matériau à l'exemple de réalisation particulier du système ancillaire chirurgical sans pour autant sortir du cadre de la présente invention et que les exemples que l'on vient de donner ne sont donc que des illustrations particulières en aucun cas limitatives des domaines d'application de l'invention.

A cette fin, la présente invention propose un système ancillaire comportant notamment un guide d'insertion apte à recevoir un ligament prothétique replié autour de son extrémité pour le pousser dans un tube fémoral, le guide d'insertion comportant une tige dont au moins une portion a une section biconcave, les concavités latérales de la portion étant aptes à recevoir le ligament prothétique allongé le long de la tige. On comprend bien qu'avec le système ancillaire selon l'invention, on peut, en toute sécurité, aisément et de manière précise et fiable insérer un ligament prothétique dans un tube fémoral préalablement foré à cet effet.

Aucun des trois documents décrits ci-dessus ne divulgue un système ancillaire comportant un tel guide d'insertion.

## Revendications

1. Système ancillaire chirurgical (1) notamment pour la plastie en fixation transversale du ligament croisé antérieur du genou, **caractérisé en ce qu'**il est pourvu d'au moins d'un premier outil chirurgical, d'un second outil chirurgical et d'une platine (2) comportant au moins :
- un stabilisateur (5) destiné à définir au moins un point d'ancrage fixe de ladite platine (2) sur le genou,
- des premiers moyens de guidage (30) d'axe longitudinal A1, aptes à recevoir au moins le premier outil chirurgical et à guider le déplacement rectiligne dudit premier outil chirurgical selon ledit axe longitudinal A1, en coulissant par rapport à ladite platine (2) fixe,
- des seconds moyens de guidage (42) d'axe transversal A3, aptes à recevoir au moins le second outil chirurgical et à guider le déplacement rectiligne dudit second outil chirurgical selon ledit axe transversal A3, en coulissant par rapport à ladite platine (2) fixe,
lesdits premiers et seconds moyens de guidage (30, 42) étant agencés pour que lesdits axes longitudinal A1 et transversal A3 soient sécants entre eux,
et **en ce que** l'un au moins desdits premier et second outils chirurgicaux est un guide d'insertion (10) apte à recevoir un ligament prothétique (200) replié autour de son extrémité pour le pousser dans un tube fémoral, ledit guide d'insertion (10) comportant une tige (100) présentant depuis une de ses extrémités une troisième section (S12) de section plate, dont l'extrémité libre est pourvue d'une découpe concave (102) apte à engager un ligament prothétique plié autour de cette découpe concave (102), ladite découpe concave (102) étant de préférence en forme de demi-lune, cette troisième portion (S12) étant suivie d'une première portion (S11) avant une section biconcave, les concavités latérales de ladite première portion (S11) et les plats de ladite troisième portion (S12) étant aptes à recevoir ledit ligament prothétique allongé le long de ladite tige (100).

2. Système ancillaire chirurgical (1) selon la revendication 1, **caractérisé en ce que** lesdits premiers et seconds moyens de guidage (30, 42) sont agencés pour que lesdits axes longitudinal A1 et transversal A3 soient sensiblement perpendiculaires entre eux et coplanaires.

3. Système ancillaire chirurgical (1) selon l'une quelconque des revendications précédentes, **caractérisé en ce que** ladite platine (2) est pourvue, en une première extrémité (2a), d'un bras latéral (3) comportant lesdits premiers moyens de guidage (30) et, en une seconde extrémité (2b), d'une tête (4) comportant lesdits seconds moyens de guidage (42).

4. Système ancillaire chirurgical (1) selon la revendication 3, **caractérisé en ce que** l'un au moins desdits tête (4), bras latéral (3) est amovible et fixé sur ladite platine (2) par des moyens d'assemblage (21, 22, 31, 32, 10).

5. Système ancillaire chirurgical (1) selon la revendication 3, **caractérisé en ce que** ladite tête (4) comporte au moins un premier et un second orifices (41, 42) respectivement d'axes transversaux A2 et A3, parallèles entre eux, ledit axe transversal A2 étant décalé longitudinalement par rapport audit axe transversal A3, ledit premier orifice (41) étant agencé pour porter ledit stabilisateur (5), ledit second orifice (42) définissant au moins en partie lesdits seconds moyens de guidage.

6. Système ancillaire chirurgical (1) selon la revendication 5, **caractérisé en ce que** ledit premier orifice (41) est prévu au delà dudit second orifice (42) par rapport à ladite seconde extrémité (2b).

7. Système ancillaire chirurgical (1) selon l'une quelconque des revendications précédentes, **caractérisé en ce qu'**il comporte des moyens de verrouillage (34, 36, 37) couplés à au moins l'un desdits premiers (30), seconds moyens de guidage (42) et agencés pour bloquer en position respectivement ledit premier, second outil chirurgical.

8. Système ancillaire chirurgical (1) selon la revendication 7, **caractérisé en ce que** lesdits moyens de verrouillage comportent au moins une rainure de guidage (30) définissant un logement parallélépipédique, **en ce que** l'un au moins desdits premier, second outils chirurgicaux a au moins une section parallélépipédique agencée pour s'emboîter dans ladite rainure de guidage (30).

9. Système ancillaire chirurgical (1) selon l'une quelconque des revendications précédentes, **caractérisé en ce que** l'un au moins desdits premier et second moyens de guidage (30, 42) est couplé à au moins une butée (7) agencée pour limiter le déplacement respectivement transversal, longitudinal dudit premier, second outil chirurgical.

10. Système ancillaire chirurgical (1) selon l'une quelconque des revendications précédentes, **caractérisé en ce que** ladite platine (2) et ledit bras latéral (3) ont chacun une forme globalement parallélépipédique et **en ce que** ledit bras latéral (3) est fixé sensiblement perpendiculaire à ladite platine (2).

## Patentansprüche

1. Chirurgisches Hilfssystem (1), insbesondere für die Ersatzplastik des vorderen Kreuzbandes des Knies mit transversaler Fixation, **dadurch gekennzeichnet, dass** es mit mindestens einem ersten chirurgischen Instrument ausgestattet ist, mit einem zweiten chirurgischen Instrument und einer Platine (2), umfassend mindestens:
- einen Stabilisator (5), der dazu ausgelegt ist, mindestens einen festen Verankerungspunkt der Platine (2) auf dem Knie zu definieren,
- erste Führungsmittel (30) mit einer Längsachse A1, die dazu geeignet sind, mindestens das erste chirurgische Instrument aufzunehmen und die geradlinige Verschiebung des ersten chirurgischen Instruments entlang der Längsachse A1, gleitend mit Bezug auf die feste Platine (2) zu führen,
- zweite Führungsmittel (42) mit einer Querachse A3, die dazu geeignet sind, mindestens das zweite chirurgische Instrument aufzunehmen und die geradlinige Verschiebung des zweiten chirurgischen Instruments entlang der Querachse A3 gleitend mit Bezug auf die feste Platine (2) zu führen,
wobei das erste und das zweite Führungsmittel (30, 42) angeordnet sind, damit die Längsachse A1 und die Querachse A3 sich untereinander schneiden,
und dadurch, dass mindestens eines des ersten und des zweiten chirurgischen Instruments eine Einsatzführung (10) ist, die dazu geeignet ist, ein prothetisches Band (200) aufzunehmen, das um sein Ende geschlagen ist, um es in ein Femoralrohr zu schieben, wobei die Einsatzführung (10) einen Schaft (100) aufweist, der von einem seiner Enden einen dritten Abschnitt (S12) mit einem ebenen Schnitt aufweist, dessen freies Ende mit einem konkaven Schnitt (102) ausgestattet ist, der dazu geeignet ist, in ein prothetisches Band, das um diesen konkaven Schnitt (102) geschlagen ist, einzugreifen, wobei der konkave Schnitt (102) vorzugsweise halbmondförmig ist, wobei diesem dritten Abschnitt (S12) ein erster Abschnitt (S11) folgt, der einen bikonkaven Schnitt aufweist, wobei die seitlichen Konkavitäten des ersten Abschnitts (S11) und die Flächen des dritten Abschnitts (S12) dazu geeignet sind, das verlängerte prothetische Band entlang des Schafts (100) aufzunehmen.

2. Chirurgisches Hilfssystem (1) nach Anspruch 1, **dadurch gekennzeichnet, dass** das erste und das zweite Führungsmittel (30, 42) angeordnet sind, damit die Längsachse A1 und die Querachse A3 im Wesentlichen senkrecht zueinander und koplanar sind.

3. Chirurgisches Hilfssystem (1) nach einem der vorhergehenden Ansprüche 1, **dadurch gekennzeichnet, dass** die Platine (2), an einem ersten Ende (2a) mit einem seitlichen Arm (3) ausgestattet ist, der die ersten Führungsmittel (30) umfasst, und an einem zweiten Ende (2b) mit einen Kopf (4), der die zweiten Führungsmittel (42) umfasst.

4. Chirurgisches Hilfssystem (1) nach Anspruch 3, **dadurch gekennzeichnet, dass** mindestens einer des Kopfs (4), des seitlichen Arms (3) auf der Platine (2) durch Montagemittel (21, 22, 31, 32, 10) abnehmbar und befestigt ist.

5. Chirurgisches Hilfssystem (1) nach Anspruch 3, **dadurch gekennzeichnet, dass** der Kopf (4) mindestens eine erste und eine zweite Öffnung (41, 42) aufweist, jeweils mit Querachsen A2 und A3, die parallel zueinander sind, wobei die Querachse A2 der Länge nach mit Bezug auf die Querachse A3 verschoben ist, wobei die erste Öffnung (41) angeordnet ist, um den Stabilisator (5) zu tragen, wobei die zweite Öffnung (42) mindestens teilweise die zweiten Führungsmittel definiert.

6. Chirurgisches Hilfssystem (1) nach Anspruch 5, **dadurch gekennzeichnet, dass** die erste Öffnung (41) über die zweite Öffnung (42) hinaus mit Bezug auf das zweite Ende (2b) vorgesehen ist.

7. Chirurgisches Hilfssystem (1) nach einem der vorhergehenden Ansprüche, **dadurch gekennzeichnet, dass** es Verriegelungsmittel (34, 36, 37) umfasst, die an mindestens eines des ersten (30) und zweiten Führungsmittels (42) gekoppelt und angeordnet sind, um das erste bzw. zweite chirurgische Instrument in seiner Position zu blockieren.

8. Chirurgisches Hilfssystem (1) nach Anspruch 7, **dadurch gekennzeichnet, dass** die Verriegelungsmittel mindestens eine Führungsnut (30) umfassen, die eine parallelepipedische Aufnahme definiert, dadurch, dass mindestens eines des ersten und des zweiten chirurgischen Elements mindestens einen parallelepipedischen Abschnitt aufweist, der angeordnet ist, um sich in die Führungsnut (30) einzuschieben.

9. Chirurgisches Hilfssystem (1) nach einem der vorhergehenden Ansprüche, **dadurch gekennzeichnet, dass** mindestens eines des ersten und des zweiten Führungsmittels (30, 42) an mindestens einen Anschlag (7) gekoppelt ist, der angeordnet ist, um die Quer- bzw. Längsverschiebung des ersten und des zweiten chirurgischen Instruments zu begrenzen.

10. Chirurgisches Hilfssystem (1) nach einem der vorhergehenden Ansprüche 1, **dadurch gekennzeichnet, dass** die Platine (2) und der seitliche Arm (3) jeweils eine im Wesentlichen eine parallelepipedische Form aufweisen, und dadurch, dass der seitliche Arm (3) im Wesentlichen senkrecht zu der Platine (2) befestigt ist.

## Claims

1. Ancillary surgical system (1) particularly for transverse fixation reconstruction of the anterior cruciate ligament of the knee, **characterised in that** it is provided with at least one surgical tool, a second surgical tool, and a plate (2) comprising at least:
- a stabiliser (5) intended to define at least one fixed anchoring point of said plate (2) on the knee,
- first guidance means (30) having a longitudinal axis A1, suitable for receiving at least the first surgical tool and guiding the rectilinear movement of said first surgical tool along said longitudinal axis A1, by sliding with respect to said fixed plate (2),
- second guidance means (42) having a transverse axis A3, suitable for receiving at least the second surgical tool and guiding the rectilinear movement of said second surgical tool along said transverse axis A3, by sliding with respect to said fixed plate (2),
said first and second guidance means (30, 42) being arranged so that said longitudinal A1 and transverse A3 axes are mutually secant,
and **in that** at least one of said first and second surgical tools is an insertion guide (10) suitable for receiving a prosthetic ligament (200) folded about the end thereof so as to push same into a femoral tube, said insertion guide (10) comprising a rod (100) having from one of the ends thereof a third section (S12) having a flat section, wherein the free end is provided with a concave recess (102) suitable for engaging a prosthetic ligament folded about the concave recess (102), said concave recess (102) being preferably half-moon shaped, the third portion (S12) being followed by a first portion (S11) having a biconcave section, the lateral concavities of said first portion (S11) and the flat parts of said third portion (S12) being suitable for receiving said tapered prosthetic ligament along said rod (100).

2. Ancillary surgical system (1) according to claim 1, **characterised in that** said first and second guidance means (30, 42) are arranged so that said longitudinal A1 and transverse A3 axes are substantially perpendicular with each other and coplanar.

3. Ancillary surgical system (1) according to any of the above claims, **characterised in that** said plate (2) is provided, at a first end (2a), with a lateral arm (3) comprising said first guidance means (30) and, at a second end (2b), a head (4) comprising said second guidance means (42).

4. Ancillary surgical system (1) according to claim 3, **characterised in that** at least one of said head (4), lateral arm (3) is removable and attached to said plate (2) by assembly means (21, 22, 31, 32, 10).

5. Ancillary surgical system (1) according to claim 3, **characterised in that** said head (4) comprises at least one first and one second apertures (41, 42) having transverse axes A2 and A3 respectively, which are mutually parallel, said transverse axis A2 being longitudinally offset with respect to said transverse axis A3, said first aperture (41) being arranged to carry said stabiliser (5), said second aperture (42) defining at least partly said second guidance means.

6. Ancillary surgical system (1) according to claim 5, **characterised in that** said first aperture (41) is provided after said second aperture (42) with respect to said second end (2b).

7. Ancillary surgical system (1) according to any of the above claims, **characterised in that** it comprises locking means (34, 36, 37) coupled with at least one of said first (30), second guidance means (42) and arranged to lock said first and second surgical tool in position, respectively.

8. Ancillary surgical system (1) according to claim 7, **characterised in that** said locking means comprise at least one guidance groove (30) defining a parallepipedic housing, **in that** at least one of said first and second surgical tools has at least one parallepipedic section arranged to fit into said guidance groove (30).

9. Ancillary surgical system (1) according to any of the above claims, **characterised in that** at least one of said first and second guidance means (30, 42) is
coupled with at least one abutment (7) arranged to restrict the transverse and longitudinal movement, respectively, of the first and second surgical tool.

10. Ancillary surgical system (1) according to any of the above claims, **characterised in that** said plate (2) and said lateral arm (3) each have a generally parallepipedic shape and **in that** said lateral arm (3) is attached substantially perpendicular to said plate (2).
